# EUROPEAN PATENT APPLICATION

(11) **EP 0 790 295 A2**
(43) Date of publication of application: **20.08.1997**
(21) Application number: 97300613.3
(22) Date of filing: 30.01.1997
(51) Int. Cl.: C11D 3/00, C11D 1/62

(54) **Fabric softening composition**

(30) Priority: 14.02.1996 GB 9603026
(71) Applicant: UNILEVER PLC, London EC4P 4BQ (GB); UNILEVER N.V., 3013 AL Rotterdam (NL)
(72) Inventor: Khan-Lodhi, Abid Nadim, Chester, CH2 3HJ (GB); Whaley, Christopher, Wirral, Merseyside, L63 5NS (GB)
(74) Representative: Mole, Peter Geoffrey

(57) **Abstract**

Fabric conditioning compositions having a pH of less than 5 and comprising a fabric softening compound of general formula I and exhibiting good hydrolytic stability.

(I) (R¹-CO.-O-)ₘA(-O-CO.-B-N⁺ R²R³R⁴)ₙ NX⁻

wherein X is Br,mesylate or methylsulphate
A is an m+n valent radical remaining after the removal of m+n OH groups from an aliphatic polyol having p OH groups and a C:O ratio of 1.0 to 3.0 m, n + p are integers from 1 to p-n, 1 to p-m and at least 2 respectively.
B is a C₁-C₁₁ alkylene or alkylidene group
R¹ is a C₁₀-C₄₈ alkyl or alkenyl group, at least one being C₂₂-C₄₈ or at least 2 being C₁₆-C₂₀ or at least 3 being C₁₀-C₁₄.
R²-R⁴ are independently C₁-C₄ alkyl or alkenyl groups optionally substituted/interupted by ethylene oxide or propylene oxide groups or other CO or CNO containing groups, or R² and R³ form a 5-6 atom ring.

## Description

The present invention relates to a fabric softening compound in which a biodegradable quaternary ammonium material is stabilised by specific counter ions.

Rinse added fabric softener compositions are well known. Typically such compositions contain a water insoluble quaternary ammonium fabric softening agent dispersed in water at a level of softening agent up to 7% by weight in which case the compositions are considered dilute, or at levels from 7% to 50% in which case the compositions are considered concentrates.

Hydrolytic stability is known to be a problem with some biodegradable cationic softeners. EP 0 239 910 (Procter and Gamble) discloses compositions comprising hydrolytically unstable cationic softeners in which the composition is stabilised by regulation of the pH of the composition.

EP 0 638 639 (Akzo) discloses fabric softening compounds known as polyol quats (PEQs). However, it is known that these compounds are particularly sensitive to hydrolysis when used in aqueous fabric softening compositions.

The present application is directed towards overcoming the problem of poor hydrolytic stability of polyol quats when used in aqueous formulations.

Accordingly the present invention relates to a fabric conditioning composition comprising a fabric softening compound of formula I.

(R¹-CO.-O-)ₘA (-O-CO.-B-N⁺R²R³R⁴)ₙ nX⁻ (formula I)

In which X is a bromide, methanesulphonate ("mesylate"), or methyl sulphate anion, A is an (m + n) valent radical remaining after the removal of (m + n) hydroxy groups from an aliphatic polyol having p hydroxy groups and an atomic ratio of carbon to oxygen in the range of 1.0 to 3.0 and up to 2 groups per hydroxy group selected from ethylene oxide and propylene oxide, m is an integer from 1 to p-n, n is an integer from 1 to p-m, and p is an integer of at least 2, B is an alkylene or alkylidene group containing 1 to 4 carbons atoms, R¹ is a straight or branched chain C₁₀-C₄₈ alkyl or alkenyl group, R², R³ and R⁴ are, independently from each other, straight or branched chain C₁-C₄ alkyl or alkenyl groups, wherein R¹, R², R³ and R⁴ optionally substituted by one or more functional groups and/or interrupted by at most 10 ethylene oxide and/or propylene oxide groups, or by at most two functional groups selected from
CO.O-. -O-CO.-, 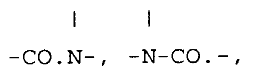 and -O-CO.O- or R² and R³ may form a ring system containing 5 or 6 atoms in the rings, with the proviso that the average compound either has at least one R¹ group having 22-48 carbon atoms, or at least two R¹ groups having 16-20 carbon atoms, or at least three R groups having 10-14 carbon atoms, and which composition has a pH of less than 5.

It is preferable if the anion (X) is bromide, more preferably mesylate.

It is preferable if the R¹ group has 16-48 carbon atoms.

It is preferable if for the compounds described above of formula I (m + n) is at least 2.

It is preferred if the compound of formula I is prepared by reacting:
a) 1 equivalent of an aliphatic polyol having p hydroxy groups,
b) m equivalents of an aliphatic carboxylic acid of the formula R¹-COOH, and
c) n equivalents of a C₁₋₄ bromocarboxylic acid or mesylated C₁₋₄ hydroxycarboxylic acid, followed by treatment with
d) n equivalents of a tertiary amine of the formula R²R³R⁴ N or n equivalents of a secondary amine of the formula R²R³NH, followed by conversion with n equivalents of a quaternizing agent.

Within the scope of the invention preference is given to compositions comprising compounds corresponding to formula I obtainable by reaction of a polyol having 2 to 8 hydroxy groups. Optimum results are obtained when the polyol is selected from ethyleneglycol, glycerol, pentaerythritol, sorbitol, glucose, saccharose, methylglucoside, and condensed derivatives thereof. The polyol may be partially etherified and/or esterified.

Good results can be obtained when the ester forming derivative of the polyol or the carboxylic acid is a natural fat, preferably a glyceride. Examples of condensed derivatives according to the invention are diglycerol, triglycerol, and dipentaerythritol.

Use may also be made of natural polyols such as starch, degraded starch, and oligomers and/or polymers containing repeating units of vinyl alcohol.

Compositions comprising compounds corresponding to formula I are obtainable by reaction of an aliphatic carboxylic acid of the formula R¹-COOH or an ester thereof. Optimum results are obtained when said acid is tallow acid (C₁₆-C₁₈) and/or at least partially hydrogenated tallow acid. Other examples of suitable quaternary ammonium compounds corresponding to the first structural formula are obtained by replacing tallow acid with, for example, coconut acid, palmitic acid, lauric acid, oleic acid, stearic acid.

The R², R³, and R⁴ groups are, independently of each other, straight or branched chain C₁-C₄ alkyl or alkenyl groups optionally substituted by one or more functional groups and/or interrupted by at most 10 ethylene oxide and/or propylene oxide groups, or by at most two functional groups selected from:
-CO.O-, O-CO.- 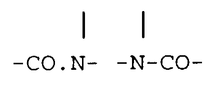 and -O-CO.O- or R² and R³ may form a ring system containing 5 or 6 atoms in the ring. Typical combinations of R²/R³/R⁴ are:
CH₃/CH₃/CH₃, C₂H₅/C₂H₅/C₂H₅ and CH₃/CH₃/CH₂CH₂OH.

The R², R³ and R⁴ groups can be introduced into the molecule by the use of appropriate tertiary amines such as trimethylamine, and triethylamine.

The R² and R³ groups can also be introduced by the use of appropriate secondary amines such as dimethylamine. The R⁴ group can then be introduced by quaternization of the thus obtained tertiary amine with halogenating agents such as methyl chloride and dimethylsulphate.

Tallow is a convenient and inexpensive source of long chain alkyl and alkenyl material.

Examples of suitable polyol quats (PEQs) as claimed by the invention are given in EP 0 638 639 (Akzo), which describes PEQs wherein the R², R³ and R⁴ groups may be independently C₁-C₄₈ alkyl or alkenyl groups.

The compositions of the invention may also contain more conventional cationic softening compounds, suitable examples of conventional softeners including amongst others those of general formula II and III. It is advantageous if the cationic softening compound is a quaternary ammonium softening compound having a solubility in water at pH 2.5 and 20°C of less than 10g/l.

It is particularly advantageous if the more conventional cationic softening compound is a quaternary ammonium compound in which at least one long chain alkyl group is connected to the quaternary ammonium group via at least one ester link.

Preferred more conventional cationic softening compounds for use with the invention are compounds of formula (II)

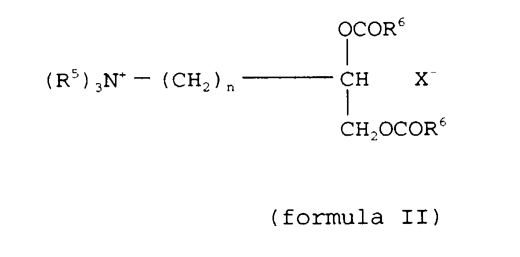

In which each R⁵ group is independently selected from C₁₋₄ alkyl, hydroxyalkyl groups or C₂₋₄ alkenyl groups; and wherein each R⁶ group is independently selected from C₈₋₂₈ alkyl or alkenyl groups; X is any suitable anion and n is o or an integer from 1 to 5.

Materials of this class and their method of preparation are described in US 4 137 180 (Lever Brothers). Analysis of such materials shows that they also comprise small amounts of the corresponding dimethylamine salt, one such salt being N,N-dimethyl-2,3-bis[hardened tallowoyloxy]-propylamine hydrochloride. Advantageously these materials comprise small amounts of the corresponding monoester as described in US 4 137 180, for example, 1-hardened tallowoyloxy-2-hydroxy-3-trimethylammonium propane chloride.

A further preferred more conventional cationic softener is represented by formula III:

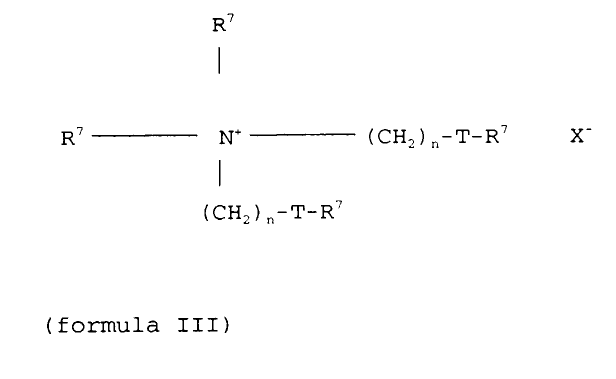

In which each R⁷ group is independently selected from C₁₋₄ alkyl, hydroxyalkyl or C₂₋₄ alkenyl groups; and wherein each R⁷ group is independently selected from C₈₋₂₈ alkyl or alkenyl groups;
T is 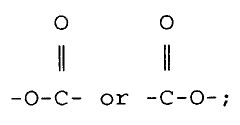 and
n is o or an integer from 1 to 5 and X⁻ is any suitable anion.

Preferably the level of ester linked quaternary ammonium compounds is at least 1% by weight of the composition, more preferably at least 3% by weight of the composition; especially interesting are concentrated compositions which comprise at least 7% of ester-linked quaternary ammonium compound. The level of ester-linked quaternary ammonium compounds preferably is from 1% to 80% by weight, more preferably from 3% to 50%, most preferably from 8% to 50%.

The composition can also contain one or more optional ingredients, selected from non-aqueous solvents, pH buffering agents, perfumes, perfume carriers, fluorescers, colorants, hydrotropes, antifoaming agents, antiredeposition agents, enzymes, optical brightening agents, opacifiers, anti-shrinking agents, anti-wrinkle agents, anti-spotting agents, germicides, fungicides, anti-oxidants, anti-corrosion agents, drape imparting agents, antistatic agents and ironing aids.

The composition may also contain nonionic fabric softening agents such as lanolin and derivatives thereof.

The softening composition may have low temperature stability improved by the addition of nonionic stabilisers. Suitable nonionic stabilisers which can be used include the condensation products of C₈-C₂₂ primary linear alcohols with 10 to 20 moles of ethylene oxide. The alcohols may be saturated or unsaturated.

Preferably, the level of nonionic stabiliser is within the range from 0.1 to 10% by weight, more preferably from 0.5 to 5% by weight, most preferably from 1 to 4% by weight. The mole ratio of the quaternary ammonium compound to the nonionic stabilising agent is within the range from 40:1 to about 1:1, preferably within the range from 18.1 to about 3:1.

The composition can also contain fatty acids, for example, C₈-C₂₄ alkyl or alkenyl monocarboxylic acids or polymers thereof. Preferably saturated fatty acids are used, in particular, hardened tallow C₁₆-C₁₈ fatty acids. Preferably the fatty acid is non-saponified, more preferably the fatty acid is free for example oleic acid, lauric acid or tallow fatty acid.

The compositions of the invention have an acidic pH of less than 5, preferably a pH from 1.3 to 3.5.

### Preparation of Softening Materials

The PEQ's were prepared by methods based on those described in EP 0 638 639 (Akzo). The bromide, mesylate, or methosulphate salts were prepared via ion exchange from the corresponding chloride materials. Such ion exchange was achieved by: (i) using an ion exchange column; or (ii) using a large excess of the required counter-ion in an appropriate solvent mix. The latter method involved use of a bromide, mesylate, or methyl sulphate salt of an alkali metal, an alkaline earth metal, a protonated amine, or a quaternary ammonium compound (mixtures of the above salts could also be used). Appropriate solvent mixes are those that allow solubilisation of the PEQ and also ionisation of the salt, or salts, of the required counter-ion. For water soluble PEQs there is an option to use only water as the solvent; for more hydrophobic PEQs an organic solvent, for example a C₁-C₄ alcohol or chloroform, is required. Further details of the types of conditions which may be employed are given in the examples.

Alternatively, direct preparation of the required PEQs may be achieved. The bromide and mesylate salts may be prepared by the method described in EP 0 638 639, substituting the C₁-C₄ chlorocarboxylic acid by a C₁-C₄ bromocarboxylic acid or mesylated hydroxycarboxylic acid. Particularly preferred materials are bromoacetic acid, mesylated glycolic acid (MeSO₂OCH₂CO₂H).

The methyl sulphate material may be directly prepared using the alternative synthetic route described in EP 0 638 639. Thus the R² and R³ groups may be introduced using a secondary amine and the R⁴ group introduced using dimethyl sulphate as the quaternisation agent. A preferred secondary amine for use in this synthetic route is N,N-dimethylamine.

In an alternative preparation of the methyl sulphate material, an N,N-di(C₁-C₄)alkylaminocarboxylic acid may be used in place of the chlorocarboxylic acids of EP 0 638 639. This results in the same intermediate as prepared by use of the secondary amine described above. Subsequent quaternisation with dimethyl sulphate gives the PEQ methyl sulphate. Preferred dialkylaminocarboxylic acids are N,N-dimethylaminoacids, in particular, N,N-dimethylglycine.

The invention will now be illustrated by the following nonlimiting examples, further modifications within the scope of the scope of the invention will be apparent to the man skilled in the art.

### Comparative Example A

Comparative Example A was a PEQ chloride of formula I, prepared from pentaerythritol, 1.7 molar equivalents of chloroacetic acid, and 2 molar equivalents of hardened tallow fatty acid, followed by reaction with 1.7 molar equivalents of trimethylamine (ex Akzo).

### Example 1

Example 1 was prepared from comparative Example A by ion exchanging the chloride counterion for bromide using an excess of sodium bromide. The PEQ chloride was dissolved in chloroform and the resulting solution stirred with a large excess of concentrated aqueous sodium bromide solution (50% w/w) for 48 hours. After this time, the chloroform layer was separated, dried over anhydrous magnesium sulphate, and then evaporated to dryness to give Example 1.

### Example 2

Example 2 was prepared from comparative Example A by ion exchanging the chloride counterion for methyl sulphate using an excess of sodium methyl sulphate. The PEQ chloride was dissolved in chloroform and warmed on a steam bath until dissolved. The resulting solution (15% w/v) was stirred with a large excess of concentrated aqueous sodium methyl sulphate solution (40% w/w) for 65 hours. After this time, the layers were separated and the chloroform layer washed with water, dried over anhydrous sodium sulphate, and then evaporated to dryness to give Example 2.

The above PEQ materials were made up as 15% w/w dispersions of active material in demineralised water by heating the mixtures at 70°C, with continuous agitation, until homogeneous. The compositions had a pH of less than 5.

The hydrolysis of the samples was measured by visual titration of the liberated strong (betaine) acid with standardised tetrabutylammonium hydroxide solution in isopropanol, using thymol blue indicator.

It was found that both the bromide and methyl sulphate materials as according to the present invention exhibited improved hydrolytic stability when compared with the chloride material from which they were prepared (see Table 1).

The samples were measured for hydrolysis after 4 weeks storage at 37°C. The % hydrolysis is expressed as the amount of strong acid (N,N,N-trimethylglycine) generated with the respect of the maximum amount of the acid which could be generated from that compound.

**Table 1:**

| **The hydrolytic stability of the Compositions** | | |
|---|---|---|
| **Example** | **Counterion** | **Hydrolytic Stability after 4 weeks @ 37 °C (expressed as % hydrolysis)** |
| Comparative A | Chloride | 35% |
| 1 | Bromide | 24% |
| 2 | Methyl sulphate | 27% |

### Example 3 to 6: Fabric Softening Compositions

The following concentrated fabric conditioning compositions further comprising conventional fabric softening compounds were prepared as described hereinabove with respect to the 15% PEQ dispersions. Details of the compositions are given in Table 2.

**Table 2:**

| **Fabric conditioning Compositions** | | | | |
|---|---|---|---|---|
| **Example** | **Example 1** | **Material of formula II**^{***1**} | **Material of formula III**^{***2**} | **Perfume** |
| 3 | 10 | 5 | - | - |
| 4 | 10 | - | 5 | 1 |
| 5 | 10 | 5 | - | - |
| 6 | 10 | - | 5 | 1 |
| All percentages are expressed as % by weight with the balance being water. | | | | |

| | | | | |
|---|---|---|---|---|
| ^{*1} 1,2 bis [hardened tallowoyloxy]-3-trimethylammonium propane chloride | | | | |
| ^{*2} di(tallowyloxyethyl)dimethyl ammonium chloride. | | | | |

## Claims

1. A fabric conditioning composition comprising a fabric softening compound of formula I:
(R¹-CO.-O-)ₘA(-O-CO.-B-N⁺R²R³R⁴)ₙ nX⁻ (formula I)
wherein X is a bromide, methanesulphonate ("mesylate"), or methyl sulphate anion, A is an (m + n) valent radical remaining after the removal of (m + n) hydroxy groups from an aliphatic polyol having p hydroxy groups and an atomic ratio of carbon to oxygen in the range of 1.0 to 3.0 and up to 2 groups per hydroxy group selected from ethylene oxide and propylene oxide, m is an integer from 1 to p-n, n is an integer from 1 to p-m, and p is an integer of at least 2,B is an alkylene or alkylidene group containing 1 to 4 carbons atoms, R¹ is a straight or branched chain C₁₀-C₄₈ alkyl or alkenyl group, R², R³ and R⁴ are, independently from each other, straight or branched chain C₁-C₄ alkyl or alkenyl groups wherein R¹, R², R³ and R⁴ are optionally substituted by one or more functional groups and/or interrupted by at most 10 ethylene oxide and/or propylene oxide groups, or by at most two functional groups selected from
CO.O-. -O-CO.-, 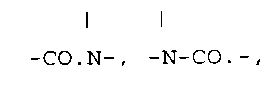 and -O-CO.O- or R² and R³ may form a ring system containing 5 or 6 atoms in the rings, with the proviso that the average compound either has at least one R¹ group having 22-48 carbon atoms, or at least two R¹ groups having 16-20 carbon atoms, or at least three R groups having 10-14 carbon atoms, and which composition has a pH of less than 5.

2. A fabric conditioning composition according to claim 1 wherein (m + n) in formula I is at least 2.

3. A fabric conditioning composition according to claim 1 wherein X is mesylate.

4. A fabric conditioning composition according to any one of claims 1, or 2 or 3 wherein the compound of formula I is prepared by reacting
a) 1 equivalent of an aliphatic polyol having p hydroxy groups
b) m equivalents of an aliphatic carboxylic acid of the formula R¹-COOH, and
c) n equivalents of a C₁₋₄ bromocarboxylic acid or mesylated hydroxycarboxylic acid, followed by treatment with
d) n equivalents of a tertiary amine of the formula R²R³R⁴ N or n equivalents of a secondary amine of the formula R²R³NH
followed by conversion with n equivalents of a quaternary agent.

5. A fabric conditioning composition according to claim 4, in which the polyol contains 2 to 8 hydroxy groups.

6. A fabric conditioning composition according to claim 5, in which the polyol is selected from the group consisting of ethylene glycol, glycerol, pentaerythritol, sorbitol, glucose, saccharose, methyl glucoside, and condensed derivatives thereof.

7. A fabric conditioning composition according to claim 6 in which the polyol is selected from the group consisting of diglycerol, triglycerol, and dipentaerythritol.

8. A fabric conditioning composition according to claim 4 in which the polyol is selected from the group consisting of natural polyols such as starch, degraded starch and oligomers and polymers containing repeating units of vinyl alcohol.

9. A fabric conditioning composition according to Claim 4 in which the compound of general formula I has an R¹ group of C₁₆-C₄₈.

10. A fabric conditioning composition according to claim 9 in which the aliphatic acid of the formula R¹-COOH is tallow acid and/or at least partially hydrogenated tallow acid.

11. A fabric conditioning composition according to claim 4 in which the tertiary amine is trimethylamine.

12. A fabric conditioning composition according to Claim 4 in which the anion is bromide, R¹ in a C₁₆-C₁₈ alkyl group, and R², R³ and R⁴ groups are C₁ groups.

13. A fabric conditioning composition according to Claim 4 in which the anion is methyl sulphate, R¹ is a C₁₆-C₁₈ alkyl group and R², R³ and R⁴ are C₁ groups.

14. A fabric conditioning composition according to any preceding claim which has a pH of from 1.3 to 3.5.
